# FASCICULE DE BREVET EUROPEEN

(11) **EP 3 440 040 B1**
(45) Date de publication et mention de la délivrance du brevet: **08.01.2020**
(21) Numéro de dépôt: 17717778.9
(22) Date de dépôt: 20.03.2017
(51) Int. Cl.: C07C 17/23, C07C 17/357, C07C 17/21, C07C 21/20, C07C 19/08

(54) **PROCÉDÉ DE PRÉPARATION DE L'HEXAFLUOROBUTADIÈNE**
VERFAHREN ZUR HERSTELLUNG VON HEXAFLUORBUTADIEN
METHOD FOR THE PREPARATION OF HEXAFLUOROBUTADIENE

(30) Priorité: 04.04.2016 FR 1652921
(43) Date de publication de la demande: 13.02.2019
(73) Titulaire: Arkema France, 92700 Colombes (FR)
(72) Inventeur: SCHMIDT, Grégory, 69700 Saint Andéol Le Château (FR); TEISSIER, Rémy, 69340 Francheville (FR)
(74) Mandataire: Leca, François Michel
(86) Numéro de dépôt international: PCT/FR2017/050635
(87) Numéro de publication internationale: WO 2017/174889

(56) Documents cités:
- US-A- 2 844 636
- TOM N. P. BOSMA ET AL: "Comparison of Reductive Dechlorination of Hexachloro-1,3-butadiene in Rhine Sediment and Model Systems with Hydroxocobalamin", ENVIRONMENTAL SCIENCE & TECHNOLOGY, vol. 28, no. 6, 1 juin 1994 (1994-06-01), pages 1124-1128, XP055321311, US ISSN: 0013-936X, DOI: 10.1021/es00055a023

## Description

### Domaine technique

L'invention concerne un procédé de préparation de l'hexafluorobutadiène. En particulier, l'invention se rapporte à la préparation de l'hexafluorobutadiène à partir de l'hexachlorobutadiène.

### Arrière-plan technologique de l'invention

Les composés fluorés ont un fort potentiel dans de nombreux domaines d'applications. Cependant, l'utilisation de nombreux composés est limitée à cause de leur méthode de préparation parfois couteuse et/ou difficile à mettre en œuvre.

Par exemple, l'hexafluorobutadiène est utilisé dans la gravure de composés électroniques. Sa préparation est réalisée par divers procédés faisant appel aux réactions couplage en C₂ au fluor F₂. Les produits de départ sont souvent des composés organiques fluorés contenant un ou plusieurs atomes d'un autre halogène. On connait notamment par US 8,536,387 la préparation de l'hexafluorobutadiène par couplage du trichloroethylène en présence de fluor F₂ suivie d'une série d'étapes alternant la déhydrochloration et la fluoration par F₂. Ce type de réaction tend à générer de nombreux produits secondaires ce qui diminue le rendement global du procédé.

On connaît également par GB 798,407 la préparation du l'hexafluorobutadiène en quatre étapes (1) dimérisation thermique du 1,2-dichloro-1,2-difluoroethylene en 1,3,4,4-tetrachloro-1,2,3,4-tetrafluorobutene-1, (2) chloration du 1,3,4,4-tetrachloro-1,2,3,4-tetrafluorobutene-1 en 1,1,2,3,4,4-hexachloro-1,2,3,4-tetrafluorobutane, (3) fluoration du 1,1,2,3,4,4-hexachloro-1,2,3,4-tetrafluorobutane en 1,2,3,4-tetrachloro-1,1,2,3,4,4-hexafluorobuane, et (4) dehydrochloration du 1,2,3,4-tetrachloro-1,1,2,3,4,4-hexafluorobuane en hexafluorobutadiene.

Une autre voie de synthèse consiste à mettre en œuvre des réactions de fluoration de l'hexachlorobutadiene en phase liquide. Cependant, ces réactions ne permettent pas la fluoration totale de l'hexachlorobutadiene pour former l'hexafluorobutadiène. On connait notamment par US 3,287,425 décrit la fluoration de l'hexachlorobutadiene en présence de fluorure de potassium pour former un mélange de 2,2-dichloroperfluoropropane et de 2-chloro-2-hydroperfluoropropane.

Le document US 2,844,636 décrit un procédé de préparation du 1,1,2,3,4,4-hexafluorobutane, des dérivés chlorés et bromés de celui-ci et du perfluorobutadiène.

Il existe donc encore un besoin pour permettre la préparation de l'hexafluorobutadiène par des réactions sélectives et abordables.

### Résumé de l'invention

Selon un premier aspect, la présente invention fournit un procédé de préparation de l'hexafluorobutadiène comprenant les étapes de :
a) Hydrodéchloration de l'hexachlorobutadiène pour former un premier flux comprenant du 1,2,3,4-tetrachlorobutadiène et optionnellement de l'hexachlorobutadiène n'ayant pas réagi,
b) Fluoration dudit premier flux comprenant du 1,2,3,4-tetrachlorobutadiène obtenu à l'étape a) pour former un second flux comprenant du 1,1,2,3,4,4-hexafluorobutane,
c) Déhydrogénation dudit second flux comprenant du 1,1,2,3,4,4-hexafluorobutane pour former un troisième flux comprenant de l'hexafluorobutadiène.

Selon un mode de réalisation préféré, l'étape b) de fluoration peut être réalisée en présence d'un agent de fluoration de formule A^{x+}Fₓ dans laquelle A est un cation et F désigne un ion fluorure F⁻.

De préférence, l'étape b) de fluoration peut être réalisée en présence d'un agent de fluoration de formule A^{x+}Fₓ dans laquelle A est H, Li, Na, K, Rb, Cs, Mg, Ca, Sr ou Ba ; et x est 1 ou 2.

Selon un mode de réalisation préféré, l'étape b) de fluoration peut être effectuée, en phase liquide, en présence d'un solvant polaire aprotique et d'un agent de fluoration de formule A^{x+}Fₓ dans laquelle A est Li, Na, K, Mg ou Ca ; et x est 1 ou 2 ; avantageusement le solvant polaire aprotique est choisi parmi le groupe consistant en un éther, un amide, une amine, un sulfoxide, une cétone, un nitrile ou un ester.

Selon un mode de réalisation préféré, le solvant organique polaire aprotique peut avoir un point d'ébullition supérieur à 100°C à pression atmosphérique.

Selon un autre mode de réalisation préféré, l'étape de fluoration b) peut être effectuée en phase gazeuse en présence d'un catalyseur et de HF.

Selon un autre mode de réalisation préféré, l'étape de fluoration b) peut être effectuée en phase liquide en présence de HF, avantageusement en présence d'un catalyseur à base d'halogénures de métaux ou d'un liquide ionique.

Selon un mode de réalisation préféré, de l'acide chlorhydrique est également produit pendant l'étape de fluoration ; ledit second flux produit à l'étape b) comprenant du 1,1,2,3,4,4-hexafluorobutane, de l'acide chlorhydrique et éventuellement de l'agent de fluoration n'ayant pas réagi ; avantageusement le second flux obtenu à l'étape b) est séparé en un premier courant comprenant l'acide chlorhydrique et éventuellement l'agent de fluoration n'ayant pas réagi et en un second courant comprenant le 1,1,2,3,4,4-hexafluorobutane. Dans ce cas, ledit second courant comprenant le 1,1,2,3,4,4-hexafluorobutane correspond audit second flux comprenant le 1,1,2,3,4,4-hexafluorobutane mentionné à l'étape c) du présent procédé.

Selon un mode de réalisation préféré, l'hexafluorobutadiène formé à l'étape c) peut être récupéré et soumis à une étape de distillation.

Selon un mode de réalisation préféré, l'étape a) peut être mise en œuvre en présence d'hydrogène.

Selon un mode de réalisation, l'étape a) peut être mise en œuvre en phase gazeuse à une température supérieure au point d'ébullition de l'hexachlorobutadiène.

Selon un mode de réalisation préféré, l'étape c) peut être mise en œuvre par i) chloration dudit second flux comprenant du 1,1,2,3,4,4-hexafluorobutane puis ii) déchloration du produit obtenu en i) en présence d'un réactif métallique comprenant du zinc. De préférence, le produit obtenu en i) est le 1,2,3,4-tetrachloroperfluorobutane.

### Description détaillée de l'invention

Selon un premier aspect, l'invention fournit un procédé de préparation de l'hexafluorobutadiène. Ledit procédé comprend les étapes successives d'hydrodéchloration, de fluoration et de déhydrogénation. Plus particulièrement, ledit procédé comprend les étapes de :
a) Hydrodéchloration de l'hexachlorobutadiène pour former un premier flux comprenant du 1,2,3,4-tetrachlorobutadiène et optionnellement de l'hexachlorobutadiène n'ayant pas réagi,
b) Fluoration dudit premier flux comprenant du 1,2,3,4-tetrachlorobutadiène obtenu à l'étape a) pour former un second flux comprenant du 1,1,2,3,4,4-hexafluorobutane,
c) Déhydrogénation dudit second flux comprenant du 1,1,2,3,4,4-hexafluorobutane pour former un troisième flux comprenant de l'hexafluorobutadiène.

Chaque étape du présent procédé peut être effectuée dans un ou plusieurs réacteurs disposés en série. Le présent procédé peut être effectué de manière continue ou discontinue. Lorsqu'un catalyseur est utilisé dans une des étapes du procédé, ledit catalyseur peut être régénéré en alternance avec la mise en œuvre de l'étape du procédé concernée.

Selon un mode de réalisation préféré, la réaction d'hydrodéchloration de l'hexachlorobutadiène réalisée à l'étape a) est mise en œuvre en présence d'hydrogène. De préférence, l'hydrogène est introduit en quantité stœchiométrique ou surstœchiométrique.

Selon un mode de réalisation préféré, l'étape a) peut être mise en œuvre en phase gazeuse ou en phase liquide. Lorsque l'étape a) est mise en œuvre en phase gazeuse, celle-ci peut être effectuée à une température supérieure au point d'ébullition de l'hexachlorobutadiène. En particulier, lorsque l'étape a) est mise en œuvre en phase gazeuse, celle-ci peut être effectuée à une température supérieure à 220°C, de préférence supérieure à 250°C, en particulier supérieure à 270°C. En particulier, la température à laquelle l'étape a) est réalisée est comprise entre 220°C et 400°C, de préférence entre 240°C et 370°C.

De préférence, l'étape a) du présent procédé peut être mise en œuvre en présence d'un catalyseur supporté ou non, ledit catalyseur comprenant un métal de transition choisi parmi les métaux des colonnes 4 à 11 du tableau périodique. De préférence, ledit catalyseur comprend un métal de transition choisi parmi le nickel, le cobalt, le fer, le ruthénium, le rhodium, le palladium, le rhénium, le platine, le cuivre, l'iridium, l'osmium, le vanadium, le chrome, le molybdène, le tungstène, le zinc ou un mélange de ceux-ci. En particulier, ledit catalyseur comprend un métal de transition choisi parmi le nickel, le cobalt, le fer, le ruthénium, le rhodium, le palladium, le rhénium, le platine, le cuivre et le zinc ou un mélange de ceux-ci.

De préférence, le catalyseur peut être supporté. Le support peut être du charbon actif, de la silice ou être constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838. De préférence, le support peut être du charbon actif, de l'alumine, de la silice ou de l'aluminosilicate.

De préférence, le support peut avoir une surface spécifique supérieure à 50m²/g, avantageusement supérieure à 75 m²/g, de préférence supérieure à 100 m²/g, en particulier supérieure à 125m²/g, et plus particulièrement supérieure à 150m²/g.

De préférence, le catalyseur utilisé pour la réaction d'hydrodéchloration peut être sous forme solide, de préférence sous forme de pastille. En particulier, le catalyseur peut être déposé sur un lit fixe.

Selon un mode de réalisation particulier, ledit catalyseur utilisé à l'étape a) du présent procédé comprend du nickel, du cobalt, du fer, du ruthénium, du rhodium, du palladium, du rhénium, du platine, du cuivre ou du zinc ou un mélange de ceux-ci dispersé sur un support de surface spécifique supérieure à 150m²/g, en particulier le support est de la silice, de l'alumine ou un aluminosilicate. De manière privilégiée, ledit catalyseur utilisé à l'étape a) du présent procédé comprend du nickel, du cobalt, du fer, du palladium, du platine ou du cuivre ou un mélange de ceux-ci dispersé sur un support de surface spécifique supérieure à 150m²/g, en particulier le support est de la silice, de l'alumine ou un aluminosilicate.

Le teneur en poids du métal dans le catalyseur est compris entre 0,01% et 5% en poids, avantageusement entre 0,05% et 3% en poids, de préférence entre 0,1% et 2% en poids.

Le temps de contact entre le catalyseur et les réactifs de l'étape a) est compris entre 2s et 60s, avantageusement entre 2s et 45s, de préférence entre 5s et 30s et en particulier entre 5s et 20s.

L'étape a) du présent procédé peut être mise en œuvre en présence d'un gaz inerte, avantageusement en présence d'azote, d'hélium ou d'argon, de préférence en présence d'azote.

De préférence, l'hexachlorobutadiène est vaporisé préalablement à son introduction dans le réacteur d'hydrodéchloration. En particulier, l'hexachlorobutadiène, après vaporisation, est mélangé avec de l'hydrogène et optionnellement avec un gaz inerte. De préférence, l'hexachlorobutadiène, après vaporisation, est mélangé avec de l'hydrogène et de l'azote. Ce mélange peut être effectué avant l'introduction dans le réacteur d'hydrodéchloration ou le mélange peut être effectué directement dans le réacteur d'hydrodéchloration.

De préférence, l'hydrogène est introduit avec un débit compris entre 0,001 moles/h et 5 moles/h, avantageusement entre 0,005 moles/h et 0,5 moles/h. De préférence, l'hexachlorobutadiène est introduit avec un débit compris entre 0,001 moles/h et 5 moles/h, avantageusement entre 0,005 moles/h et 0,5 moles/h.

Selon un mode de réalisation préféré, outre le 1,2,3,4-tetrachlorobutadiène, ledit premier flux obtenu à l'étape a) comprend HCl, de l'hydrogène n'ayant pas réagi et optionnellement de l'azote.

Avantageusement, une étape de séparation du flux de produits obtenu à l'étape a) est mise en œuvre avant l'étape b).

De préférence ladite étape de séparation comprend la formation d'un courant comprend HCl, l'hydrogène n'ayant pas réagi et optionnellement de l'azote et d'un second courant comprend le 1,2,3,4-tetrachlorobutadiène; en particulier ce second courant est utilisé à l'étape b).

Alternativement, l'étape a) du présent procédé peut être réalisée en phase liquide. De préférence, l'étape a) peut être réalisée en présence d'un solvant. Dans ce mode de réalisation, l'étape a) du présent procédé est effectuée à une température comprise entre 70°C à 200°C. Avantageusement, le solvant utilisé peut être un solvant qui ne réagit pas avec l'hydrogène dans les conditions opératoires de l'étape a) lorsque celle-ci est réalisée en phase liquide. De préférence, le solvant peut être un éther, un ester ou un hydrocarbure.

L'hydrocarbure peut être un alcane de formule CₙH₂ₙ₊₂ dans laquelle n est un nombre entier de 5 à 20. Le terme « éther » se réfère à un composé de formule R¹-O-R² dans laquelle R1 et R2 représentent indépendamment l'un de l'autre un alkyl en C₁-C₂₀, de préférence en C₂-C₁₅, en particulier en C₃-C₁₀ ; un aryl en C₆-C₁₈ ; un cycloalkyl en C₃-C₂₀. Le terme « alkyle » désigne un radical monovalent issu d'un alcane, linéaire ou branché, comprenant de 1 à 20 atomes de carbone. Le terme « cycloalkyle » désigne un radical monovalent issu d'un cycloalcane comprenant de 3 à 20 atomes de carbone. Le terme « aryle » désigne un radical monovalent issu d'un arène comprenant de 6 à 18 atomes de carbone. Le terme « ester » se réfère à un composé de formule R¹-C(O)-O-R² dans laquelle R1 et R2 représentent indépendamment l'un de l'autre un alkyl en C₁-C₂₀, de préférence en C₂-C₁₅, en particulier en C₃-C₁₀ ; un aryle en C₆-C₁₈ ; un cycloalkyle en C₃-C₂₀.

Dans ce mode de réalisation alternatif, l'hydrogène est introduit en quantité surstœchiométrique.

De préférence, la réaction est menée pendant une durée comprise entre 30 minutes et 10 heures, avantageusement entre 45 minutes et 7 heures, en particulier entre 1 heure et 4 heures.

De préférence, dans ce mode réalisation alternatif, l'étape a) est effectuée dans un autoclave sous agitation. A la fin de l'étape a), l'autoclave est décomprimé. Un flux d'azote peut être introduit dans l'autoclave avant récupération du mélange réactionnel. Ce dernier peut être concentré avant de mettre en œuvre l'étape b) du présent procédé avec celui-ci.

L'étape b) du présent procédé est une réaction de fluoration qui peut être réalisée en phase gazeuse ou en phase liquide.

Selon un premier mode de réalisation, l'étape de fluoration b) est réalisée en phase gazeuse.

Avantageusement, l'étape b) de fluoration peut être réalisée en présence d'un agent de fluoration de formule A^{x+}Fₓ dans laquelle A est un cation et F désigne un ion fluorure F⁻. De préférence, l'étape b) de fluoration est réalisée en présence d'un agent de fluoration de formule A^{x+}Fₓ dans laquelle A est H, Li, Na, K, Rb, Cs, Mg, Ca, Sr ou Ba ; et x est 1 ou 2.

La réaction peut être conduite en présence d'un catalyseur solide supporté ou non. Le catalyseur peut être un acide de Lewis. Par exemple, le catalyseur est à base d'un métal comprenant un oxyde de métal de transition ou un halogénure ou un oxyhalogénure d'un tel métal ou le catalyseur est un acide de Lewis à base d'un métal comprenant l'aluminium, le titane, le niobium, le tantale, l'étain, l'antimoine, le nickel, le zinc, l'hafnium, le zirconium ou le fer.

De préférence, l'agent de fluoration est HF. Le rapport molaire entre l'acide fluorhydrique et ledit premier flux comprenant du 1,2,3,4-tetrachlorobutadiène obtenu à l'étape a) est compris entre 1 et 100, de préférence entre 2 et 70, en particulier entre 5 et 55.

Selon un mode de réalisation préféré, la température à laquelle l'étape b) de fluoration est effectuée est comprise entre 100°C et 450°C, avantageusement entre 125°C et 400°C, de préférence entre 150°C et 380C°.

Selon un mode de réalisation préféré, l'étape b) de fluoration en phase gazeuse est effectuée à une pression comprise entre 1 et 20 barg, avantageusement entre 2 et 15 barg, de préférence entre 3 et 10 barg.

De préférence, l'étape b) du présent procédé peut être mise en œuvre à une température de 150°C à 380°C, à une pression de 3 à 10 barg, et avec un rapport molaire entre le HF et comprenant du 1,2,3,4-tetrachlorobutadiène obtenu à l'étape a) de 5 à 55.

Selon un mode de réalisation préféré, de l'acide chlorhydrique est également produit pendant l'étape b) de fluoration. Avantageusement, l'acide chlorhydrique est séparé dudit second flux comprenant du 1,1,2,3,4,4-hexafluorobutane, de préférence cette séparation est effectuée préalablement à l'étape c), c'est-à-dire préalablement à la mise en œuvre de la déhydrogénation dudit second flux comprenant du 1,1,2,3,4,4-hexafluorobutane obtenu à l'étape b) de fluoration.

Selon un mode de réalisation particulier, lorsque l'agent de fluoration est l'acide fluorhydrique, ledit second flux comprend HF, HCl et le 1,1,2,3,4,4-hexafluorobutane. Avantageusement, les produits issus de l'étape b) sont séparés en un premier courant comprenant HF et HCl et en un second courant comprenant le 1,1,2,3,4,4-hexafluorobutane, de préférence préalablement à l'étape c). Ainsi, ledit second courant peut être ledit second flux comprenant le 1,1,2,3,4,4-hexafluorobutane mis en œuvre à l'étape c) du présent procédé.

Selon un second mode de réalisation, l'étape b) de fluoration peut être effectuée en phase liquide.

Selon un mode de réalisation préféré, l'étape de fluoration b) est effectuée, en phase liquide, en présence de HF. Avantageusement, cette étape est mise en œuvre en présence d'un catalyseur à base d'halogénures de métaux ou d'un liquide ionique.

Selon un mode de réalisation préféré, ledit catalyseur peut être à base d'halogénures de métaux, dans lequel le métal est choisi parmi le groupe consistant en l'aluminium, le titane, le niobium, le tantale, l'étain, l'antimoine, le nickel, le zinc, l'hafnium, le zirconium ou le fer.

Selon un mode de réalisation préféré, ledit catalyseur peut être un liquide ionique. Le liquide ionique peut être issu d'acide de Lewis à base d'un métal choisi parmi le groupe consistant en l'aluminium, le titane, le niobium, le tantale, l'étain, l'antimoine, le nickel, le zinc, l'hafnium, le zirconium ou le fer. Le liquide ionique est un sel aqueux ayant un caractère ionique, et qui est liquide à des températures modérées, de préférence à des températures inférieures à 120°C.

De préférence, le liquide ionique est obtenu par réaction entre au moins un halogénure ou un oxyhalogénure de métaux choisi parmi le groupe consistant en l'aluminium, le titane, le niobium, le tantale, l'étain, l'antimoine, le nickel, le zinc, l'hafnium, le zirconium ou le fer avec un sel de formule générale Y⁺A⁻ dans lequel A⁻ représente un anion de type halogénure ou un anion du type hexafluorure d'antimoine, et Y⁺ représente un cation de type ammonium quaternaire, un cation de type phosphonium quaternaire ou un cation du type sulfonium ternaire. Le terme « halogénure » se réfère à un anion chlorure, bromure, iodure ou fluorure ou un mélange de ceux-ci. Peuvent être mentionnés plus particulièrement les chlorures, fluorures ou chlorofluorures des formules suivantes :
TiClₓF_{y} avec x+y = 4 et 0 ≤ x < 4
TaClₓF_{y} avec x+y = 5 et 0 < x ≤ 5
NbClₓF_{y} avec x+y = 5 et 0 < x < 5
SnClₓF_{y} avec x+y = 4 et 1 < x < 4
SbClₓF_{y} avec x+y = 5 et 0 < x < 5

Comme exemples de tels composés, on peut mentionner les composés suivants : TiCl₄, TiF₄, TaCl₅, TaF₅, NbCl₅, NbF₅, SnCl₄, SnF₄, SbCl₅, SbCl₄F, SbCl₃F₂, SbCl₂F₃, SbClF , SbF₅ et leurs mélanges. Sont préférentiellement utilisés les composés suivants : TiCl₄, SnCl₄, TaCl₅+TaF₅, NbCl₅+NbF₅, SbCl₅, SbFCl₄, SbF₂Cl₃, SbF₃Cl₂, SbF₄Cl, SbF et SbCl₅+SbF₅. Sont plus particulièrement préférés les composés antimoniés. Comme exemples d'acides de Lewis oxyhalogénés, utilisables selon l'invention, on peut mentionner TiOCl₂, TiOF₂, SnOCl₂, SnOF₂ et SbOClₓF_{y} (x+y=3).

Dans le sel Y⁺A⁻, le cation Y⁺ peut répondre à l'une des formules générales suivantes : R¹R²R³R⁴N⁺, R¹R²R³R⁴P⁺, ou R¹R²R³S⁺ dans lesquelles les substituants R¹ à R⁴, identiques ou différents, désignent indépendamment les uns des autres un groupement hydrocarbyle, chlorohydrocarbyle, fluorohydrocarbyle, chlorofluorohydrocarbyle ou fluorocarbyle ayant de 1 à 10 atomes de carbone, saturé ou non, cyclique ou non, ou aromatique, l'un ou plusieurs de ces groupements pouvant également contenir un ou plusieurs hétéroatomes tels que N, P, S ou O.

Le cation ammonium, phosphonium ou sulfonium Y⁺ peut également faire partie d'un hétérocycle saturé ou non, ou aromatique ayant de 1 à 3 atomes d'azote, de phosphore ou de soufre, et répondre à l'une ou l'autre des formules générales suivantes : 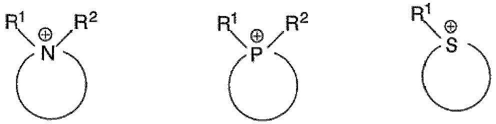 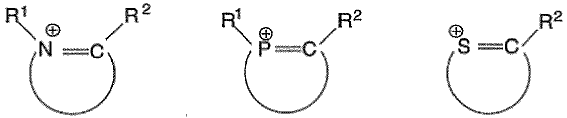 dans lesquelles R¹ et R² sont tels que définis précédemment.

On ne sortirait pas du cadre de la présente invention en utilisant un sel contenant 2 ou 3 sites ammonium, phosphonium ou sulfonium dans leur formule. Comme exemples de sels Y⁺A⁻ on peut mentionner les chlorures et fluorures de tétraalkylammonium, les chlorures et fluorures de tétraalkylphosphonium, et chlorures et fluorures de trialkylsulfonium, les chlorures et fluorures d'alkylpyridinium, les chlorures, fluorures et bromures de dialkylimidazolium, et les chlorures et fluorures de trialkylimidazolium. Sont plus particulièrement appréciés le fluorure ou le chlorure de triméthylsulfonium, le chlorure ou le fluorure de N-éthyl-pyridinium, le chlorure ou le fluorure de N-butyl-pyridinium, le chlorure ou le fluorure de 1-éthyl-3-méthyl-imidazolium, et le chlorure ou fluorure de 1-butyl-3-méthyl-imidazolium.

Les liquides ioniques selon l'invention peuvent être préparés de façon connue en soi en mélangeant de manière appropriée l'acide de Lewis halogéné ou oxyhalogéné et le sel organique Y⁺A⁻ dans un rapport molaire qui peut aller de 0,5:1 à 3,5:1, de préférence de 1:1 à 2,5:1 et plus préférentiellement 1:1 à 2:1. Un rapport molaire strictement supérieur à 1:1 est particulièrement recommandé si l'on désire obtenir un liquide ionique acide.

De préférence, le liquide ionique peut être à base d'antimoine. Par exemple, le liquide ionique peut être le produit de la réaction entre le pentachlorure d'antimoine avec le chlorure d'éthylméthylimidazolium, de sorte à obtenir le catalyseur emim⁺Sb₂F₁₁⁻.

Le mélange peut être réalisé dans un réacteur de type autoclave, éventuellement refroidi pour limiter l'exothermie de la réaction. On peut également contrôler cette exothermie en ajoutant progressivement l'un des réactifs à l'autre.

La quantité molaire d'HF sur la quantité molaire dudit premier flux comprenant du 1,2,3,4-tetrachlorobutadiène obtenu à l'étape a) est comprise entre 2 et 50 et préférentiellement entre 10 et 30. La durée de la réaction nécessaire qui dépend de la quantité des réactifs mise en jeu au départ et des différents paramètres opératoires, peut être facilement connue expérimentalement.

De préférence, l'étape b) de fluoration en phase liquide en présence de HF est effectuée à une pression de 5 à 25 barg.

Le matériau du réacteur de fluoration doit permettre de travailler dans les conditions de pression et de température définies précédemment. Il doit également résister à la corrosion qu'engendre le fluorure d'hydrogène. Ainsi, l'acier inoxydable ou des alliages type MONEL, INCONEL ou HASTELLOY sont particulièrement indiqués.

Selon un troisième mode de réalisation, l'étape b) de fluoration dudit premier flux comprenant du 1,2,3,4-tetrachlorobutadiène obtenu à l'étape a) peut être effectuée en présence d'un solvant polaire aprotique.

Avantageusement, le solvant polaire aprotique est choisi parmi le groupe consistant en un éther, un amide, une amine, un sulfoxide, une cétone, un nitrile ou un ester.

De préférence, le solvant organique polaire aprotique est choisi parmi le groupe consistant en 1,3-dioxane, 1,4-dioxane, 1,3,5-trioxane, tetrahydrofurane, 1,2-dimethoxyéthane, dimethylsulfoxyde, diéthylsulfoxyde, N-methylpyrrolidone, diméthylformamide, diméthylacétamide, acétate d'éthyle, acétone, propanone, 2-pentanone, butanone, n-butylacétate, triéthylamine, pyridine et acétonitrile.

En particulier, le solvant organique polaire aprotique a un point d'ébullition supérieur à 100°C à pression atmosphérique. De préférence, le solvant organique polaire aprotique est 1,3-dioxane, 1,4-dioxane, 1,3,5-trioxane, N-methylpyrrolidone, diméthylformamide, diméthylacétamide, propanone, 2-pentanone, butanone, dimethylsulfoxyde, diéthylsulfoxyde.

Dans ce troisième mode de réalisation, l'étape b) de fluoration est effectuée en présence d'un agent de fluoration de formule A^{x+}Fₓ dans laquelle A est un cation et F désigne un ion fluorure F⁻. De préférence, l'étape b) de fluoration est réalisée en présence d'un agent de fluoration de formule A^{x+}Fₓ dans laquelle A est H, Li, Na, K, Rb, Cs, Mg, Ca, Sr ou Ba ; et x est 1 ou 2. En particulier, l'étape b) de fluoration est réalisée en présence d'un agent de fluoration de formule A^{x+}Fₓ dans laquelle A est Li, Na, K, Mg ou Ca ; et x est 1 ou 2.

De préférence, le ratio molaire entre l'agent de fluoration et ledit premier flux comprenant du 1,2,3,4-tetrachlorobutadiène est inférieur à 8,7, avantageusement inférieur à 8,4, de préférence inférieur à 7,8, en particulier inférieur à 7,5, plus particulièrement inférieur à 6,9, de manière privilégiée inférieur à 6,6.

L'étape b) peut être effectuée pour une durée comprise entre 1 heure et 10 heures, avantageusement de 2 à 6 heures.

Avantageusement, l'étape b) peut être effectuée au reflux du solvant.

De préférence, l'étape b) est mise en œuvre, selon ce troisième mode de réalisation à une pression comprise 0 et 3 barg.

Selon un mode de réalisation préféré, le second flux comprenant du 1,1,2,3,4,4-hexafluorobutane obtenu à l'étape b), selon l'un quelconque des modes de réalisation, peut être concentré et/ou distillé. Par exemple, le second flux comprenant du 1,1,2,3,4,4-hexafluorobutane peut être distillé à pression atmosphérique entre 63°C et 65°C.

Selon l'étape c) du présent procédé, ledit second flux comprenant du 1,1,2,3,4,4-hexafluorobutane et obtenu à l'étape b) est soumis à une réaction de déhydrogénation.

Selon un mode de réalisation préféré, l'étape c) est mise en œuvre par i) chloration dudit second flux comprenant du 1,1,2,3,4,4-hexafluorobutane puis ii) déchloration du produit obtenu en i) en présence d'un réactif métallique comprenant du zinc, le cuivre, le manganèse ou un mélange de ceux-ci, de préférence du zinc.

De préférence, l'étape i) est réalisée en présence d'un excès de chlore. L'étape i) peut être mise en œuvre par un traitement thermique, avantageusement à une température supérieure à 80°C, de préférence à une température comprise entre 120°C et 150°C. Alternativement, l'étape i) peut être mise en œuvre par un traitement photochimique connu de l'homme du métier. Alternativement, l'étape i) peut être mise en œuvre en présence d'initiateur de radicaux tels que des peroxydes ou des composés du type azoïque, par exemple l'azoisobutyronitrile. Dans ce dernier cas, l'étape i) peut être mise en œuvre à une température comprise entre la température ambiante (par exemple 25°C) et 80°C.

De préférence, le produit obtenu à l'étape i) est une composition comprenant le 1,2,3,4-tetrachloroperfluorobutane.

De préférence, l'étape ii) est réalisée en présence d'un solvant aqueux et/ou alcoolique. Par exemple, l'étape ii) peut être réalisée en présence d'eau, d'isopropanol, de méthanol, d'éthanol ou de propanol ou un mélange de ceux-ci. En particulier, l'étape ii) peut être réalisée en présence d'eau, d'isopropanol ou un mélange de ceux-ci.

De préférence, le troisième flux comprenant l'hexafluorobutadiène formé à l'étape c) est récupéré et soumis à une étape de distillation.

### Exemples

### Exemple 1 : Hydrodéchloration de l'hexachlorobutadiène

On charge un réacteur en Inox de 500mm de long et de diamètre intérieur de 7,7mm avec un catalyseur à base de charbon actif imprégné par 8,4% de cuivre et 1,7% de palladium. La température du lit de catalyseur est portée à 270°C. Un flux d'hydrogène et d'hexachlorobutadiène est alors introduit dans le réacteur. Le débit d'hexachlorobutadiène et d'hydrogène sont respectivement de 0,022 moles/h et de 0,086 moles/h. Le temps de séjour est de 4,8 secondes. Le taux de transformation de l'hexachlorobutadiène est de 78% avec une sélectivité de 87% en 1,2,3,4-tetrachlorobutadiène. Le mélange réactionnel est récupéré par condensation et il est purifié par distillation sous vide (Point d'ébullition du 1,2,3,4-tetrachlorobutadiène = 188°C; point d'ébullition de l'hexachlorobutadiène = 210°C). Le rendement en 1,2,3,4-tetrachlorobutadiène après purification est de 60%.

### Exemple 2 : Fluoration en phase liquide du 1,2,3,4-tetrachlorobutadiène

Dans un autoclave de 1l maintenu à 0°C, on introduit 100ml de 1,2,3,4-tétrachlorobutadiène dans 400ml de HF contenant 7ml de SbCl₅. L'autoclave est muni d'une vanne de régulation de pression tarée à 20 bars. On porte peu à peu le mélange agité à 120°C. L'acide chlorhydrique produit lors de la réaction est éliminé de l'autoclave à l'aide de la vanne de régulation puis piégé selon les méthodes connues de l'homme du métier. Après deux heures à 120°C, le mélange réactionnel est refroidi et l'acide fluorhydrique en excès est éliminé par distillation. La phase organique restante est refroidie et décantée pour récupérer une composition comprenant du 1,1,2,3,4,4-hexafluorobutane. Cette dernière est distillée et on obtient le 1,1,2,3,4,4-hexafluorobutane (Point d'ébullition = 63-65°C) avec un rendement de 72%.

### Exemple 3 : Fluoration en phase gazeuse du 1,2,3,4-tetrachlorobutadiène

Dans un réacteur tubulaire de diamètre 20mm, on introduit 5g de catalyseur Ni/Cr sur AlF₃. La température du lit catalytique est portée à 360°C et un mélange de 0,28 g/h de 1,2,3,4-tétrachlorobutadiène et de 1,5 g/h de HF (soit un rapport molaire HF/1,2,3,4-tétrachlorobutadiène de 50) est mis en contact avec le catalyseur. En sortie de réacteur, l'acide fluorhydrique n'ayant pas réagi, soit 1,3 g/h, et les sous-produits de la réaction non totalement fluorés sont recyclés. Un mélange comprend de l'acide chlorhydrique et du 1,1,2,3,4,4-hexafluorobutane est obtenu. L'acide chlorhydrique et le 1,1,2,3,4,4-hexafluorobutane sont séparés pour aboutir à une composition de 1,2,3,4-tetrachlorobutadiène de grande pureté. Le rendement en 1,1,2,3,4,4-hexafluorobutane de 91% par rapport au 1,2,3,4-tétrachlorobutadiène introduit.

### Exemple 4 : Déshydrogénation du 1,1,2,3,4,4-hexafluorobutane

Dans un réacteur en verre agité de 1 litre, thermostaté par une circulation d'un thermofluide dans une enveloppe extérieure, avec une canne d'introduction gaz, ici le chlore, et relié à un piège de gaz rempli d'eau sodée, on dissout 100g de 1,1,2,3,4,4-hexafluorobutane et 10g l'azoisobutyronitrile dans 700ml de chlorobenzène. On introduit à température ambiante une quantité de chlore correspondant à environ 30% de la stœchiométrie de la réaction soit 50g. On porte le mélange agité à 50°C ; une exothermie d'une dizaine de degrés apparait vers 45°C qui correspond à la décomposition de l'amorceur radicalaire, l'azoisobutyronitrile et à l'initiation de la chloration radicalaire. On introduit alors le chlore à un débit de 75 g/h en maintenant la température du milieu inférieure à 60°C. On arrête l'introduction de chlore lorsque 150g de chlore ont été introduits. On laisse réagir après la fin de l'introduction du chlore pendant au moins 1 heure, de préférence jusqu'à l'absence d'exothermie. Le mélange réactionnel est ensuite balayé par un flux d'azote durant 30 minutes puis récupéré, lavé à l'eau. On récupère la phase organique que l'on distille pour obtenir une composition comprenant du 1,2,3,4-tetrachloroperfluorobutane (Point d'ébullition = 63°C).

Dans un réacteur agité surmonté d'un réfrigérant thermostaté relié à une bouteille en inox refroidie par de la carboglace, on introduit dans 800ml d'éthanol une quantité de zinc métallique légèrement surstœchiométrique soit ici 200g (1,55 moles de zinc). Le mélange est agité pour former une suspension de zinc et on introduit à température ambiante le 1,2,3,4-tétrachloroperfluorobutane. Le réfrigérant est maintenu à 0°C et ainsi seul l'hexafluorobutadiène traverse le réfrigérant. On récupère 72g d'hexafluorobutadiène soit un rendement de 72% par rapport au 1,1,2,3,4,4-hexafluorobutane. La pureté de l'hexafluorobutadiène est 98% en CPG.

## Revendications

1. Procédé de préparation de l'hexafluorobutadiène comprenant les étapes de :
a) hydrodéchloration de l'hexachlorobutadiène pour former un premier flux comprenant du 1,2,3,4-tetrachlorobutadiène et optionnellement de l'hexachlorobutadiène n'ayant pas réagi,
b) fluoration dudit premier flux comprenant du 1,2,3,4-tetrachlorobutadiène obtenu à l'étape a) pour former un second flux comprenant du 1,1,2,3,4,4-hexafluorobutane,
c) déhydrogénation dudit second flux comprenant du 1,1,2,3,4,4-hexafluorobutane pour former un troisième flux comprenant de l'hexafluorobutadiène.

2. Procédé selon la revendication précédente **caractérisé en ce que** l'étape b) de fluoration est réalisée en présence d'un agent de fluoration de formule A^{x+}Fₓ dans laquelle A est un cation et F désigne un ion fluorure F⁻.

3. Procédé selon la revendication précédente **caractérisé en ce que** l'étape b) de fluoration est réalisée en présence d'un agent de fluoration de formule A^{x+}Fₓ dans laquelle A est H, Li, Na, K, Rb, Cs, Mg, Ca, Sr ou Ba ; et x est 1 ou 2.

4. Procédé selon l'une quelconque des revendications précédentes **caractérisé en ce que** l'étape b) de fluoration est effectuée, en phase liquide, en présence d'un solvant polaire aprotique et d'un agent de fluoration de formule A^{x+}Fₓ dans laquelle A est Li, Na, K, Mg ou Ca ; et x est 1 ou 2 ; avantageusement le solvant polaire aprotique est choisi parmi le groupe consistant en un éther, un amide, une amine, un sulfoxyde, une cétone, un nitrile ou un ester.

5. Procédé selon la revendication précédente **caractérisé en ce que** le solvant organique polaire aprotique a un point d'ébullition supérieur à 100°C à pression atmosphérique.

6. Procédé selon l'une quelconque des revendications 1 à 3 **caractérisé en ce que** l'étape b) de fluoration est effectuée en phase gazeuse en présence d'un catalyseur et de HF.

7. Procédé selon l'une quelconque des revendications 1 à 3 **caractérisé en ce que** l'étape b) de fluoration est effectuée en phase liquide en présence de HF, avantageusement en présence d'un catalyseur à base d'halogénures de métaux ou d'un liquide ionique.

8. Procédé selon l'une quelconque des revendications précédentes **caractérisé en ce que** de l'acide chlorhydrique est également produit pendant l'étape de fluoration ; ledit second flux produit à l'étape b) comprenant du 1,1,2,3,4,4-hexafluorobutane, de l'acide chlorhydrique et éventuellement de l'agent de fluoration n'ayant pas réagi ; avantageusement le second flux obtenu à l'étape b) est séparé en un premier courant comprenant l'acide chlorhydrique et éventuellement l'agent de fluoration n'ayant pas réagi et en un second courant comprenant le 1,1,2,3,4,4-hexafluorobutane.

9. Procédé selon l'une quelconque des revendications précédentes **caractérisé en ce que** l'hexafluorobutadiène formé à l'étape c) est récupéré et soumis à une étape de distillation.

10. Procédé selon l'une quelconque des revendications précédentes **caractérisé en ce que** l'étape a) est mise en œuvre en présence d'hydrogène.

11. Procédé selon l'une quelconque des revendications précédentes **caractérisé en ce que** l'étape a) est mise en œuvre en phase gazeuse à une température supérieure au point d'ébullition de l'hexachlorobutadiène.

12. Procédé selon l'une quelconque des revendications précédentes **caractérisé en ce que** l'étape c) est mise en œuvre par i) chloration dudit second flux comprenant du 1,1,2,3,4,4-hexafluorobutane puis ii) déchloration du produit obtenu en i) en présence d'un réactif métallique comprenant du zinc.

13. Procédé selon la revendication précédente **caractérisé en ce que** le produit obtenu en i) est le 1,2,3,4-tetrachloroperfluorobutane.

## Patentansprüche

1. Verfahren zur Herstellung von Hexafluorbutadien, umfassend die folgenden Schritte:
a) Hydrodechlorierung von Hexachlorbutadien zur Herstellung eines ersten Stroms, der 1,2,3,4-Tetrachlorbutadien und gegebenenfalls nicht umgesetztes Hexachlorbutadien umfasst,
b) Fluorierung des in Schritt a) erhaltenen ersten Stroms, der 1,2,3,4-Tetrachlorbutadien umfasst, zur Herstellung eines zweiten Stroms, der 1,1,2,3,4,4-Hexafluorbutan umfasst,
c) Dehydrierung des zweiten Stroms, der 1,1,2,3,4,4-Hexafluorbutan umfasst, zur Herstellung eines dritten Stroms, der Hexafluorbutadien umfasst.

2. Verfahren nach dem vorhergehenden Anspruch, **dadurch gekennzeichnet, dass** der Fluorierungsschritt b) in Gegenwart eines Fluorierungsmittels der Formel A^{x+}Fₓ durchführt wird, worin A ein Kation ist und F ein Fluoridion F⁻ bedeutet.

3. Verfahren nach dem vorhergehenden Anspruch, **dadurch gekennzeichnet, dass** der Fluorierungsschritt b) in Gegenwart eines Fluorierungsmittels der Formel A^{x+}Fₓ durchführt wird, worin A H, Li, Na, K, Rb, Cs, Mg, Ca, Sr oder Ba ist und x 1 oder 2 ist.

4. Verfahren nach einem der vorhergehenden Ansprüche, **dadurch gekennzeichnet, dass** der Fluorierungsschritt b) in der Flüssigphase in Gegenwart eines polaren aprotischen Lösungsmittels und eines Fluorierungsmittels der Formel A^{x+}Fₓ durchführt wird, worin A Li, Na, K, Mg oder Ca ist und x 1 oder 2 ist, wobei vorteilhafterweise das polare aprotische Lösungsmittel aus der Gruppe ausgewählt ist bestehend aus einem Ether, einem Amid, einem Amin, einem Sulfoxid, einem Keton, einem Nitril oder einem Ester.

5. Verfahren nach dem vorhergehenden Anspruch, **dadurch gekennzeichnet, dass** das organische polare aprotische Lösungsmittel einen Siedepunkt über 100°C bei Atmosphärendruck hat.

6. Verfahren nach einem der Ansprüche 1 bis 3, **dadurch gekennzeichnet, dass** der Fluorierungsschritt b) in der Gasphase in Gegenwart eines Katalysators und von HF durchgeführt wird.

7. Verfahren nach einem der Ansprüche 1 bis 3, **dadurch gekennzeichnet, dass** der Fluorierungsschritt b) in der Flüssigphase in Gegenwart von HF, vorteilhafterweise in Gegenwart eines Katalysators auf der Basis von Metallhalogeniden oder einer Ionenflüssigkeit, durchgeführt wird.

8. Verfahren nach einem der vorhergehenden Ansprüche, **dadurch gekennzeichnet, dass** während des Fluorierungsschritts auch Salzsäure hergestellt wird, wobei der in Schritt b) hergestellte zweite Strom 1,1,2,3,4,4-Hexafluorbutan, Salzsäure und gegebenenfalls nicht umgesetztes Fluorierungsmittel umfasst, wobei vorteilhafterweise der in Schritt b) erhaltene zweite Strom in eine erste Strömung, die die Salzsäure und gegebenenfalls das nicht umgesetzte Fluorierungsmittel umfasst, und eine zweite Strömung, die das 1,1,2,3,4,4-Hexafluorbutan umfasst, aufgetrennt wird.

9. Verfahren nach einem der vorhergehenden Ansprüche, **dadurch gekennzeichnet, dass** das in Schritt c) hergestellte Hexafluorbutadien gewonnen und einem Destillationsschritt unterworfen wird.

10. Verfahren nach einem der vorhergehenden Ansprüche, **dadurch gekennzeichnet, dass** Schritt a) in Gegenwart von Wasserstoff durchgeführt wird.

11. Verfahren nach einem der vorhergehenden Ansprüche, **dadurch gekennzeichnet, dass** Schritt a) in der Gasphase bei einer Temperatur oberhalb des Siedepunkts von Hexachlorbutadien durchgeführt wird.

12. Verfahren nach einem der vorhergehenden Ansprüche, **dadurch gekennzeichnet, dass** Schritt c) durch i) Chlorierung des zweiten Stroms, der 1,1,2,3,4,4-Hexafluorbutan umfasst, und anschließende ii) Dechlorierung des in i) erhaltenen Produkts in Gegenwart eines Zink umfassenden Metallreaktanten durchgeführt wird.

13. Verfahren nach dem vorhergehenden Anspruch, **dadurch gekennzeichnet, dass** das in i) erhaltene Produkt 1,2,3,4-Tetrachlorperfluorbutan ist.

## Claims

1. Process for the preparation of hexafluorobutadiene comprising the stages of:
a) hydrodechlorination of hexachlorobutadiene in order to form a first stream comprising 1,2,3,4-tetrachlorobutadiene and optionally unreacted hexachlorobutadiene,
b) fluorination of said first stream comprising 1,2,3,4-tetrachlorobutadiene obtained in stage a) in order to form a second stream comprising 1,1,2,3,4,4-hexafluorobutane,
c) dehydrogenation of said second stream comprising 1,1,2,3,4,4-hexafluorobutane in order to form a third stream comprising hexafluorobutadiene.

2. Process according to the preceding claim, **characterized in that** the fluorination stage b) is carried out in the presence of a fluorinating agent of formula A^{x+}Fₓ in which A is a cation and F denotes a fluoride ion F⁻.

3. Process according to the preceding claim, **characterized in that** the fluorination stage b) is carried out in the presence of a fluorinating agent of formula A^{x+}Fₓ in which A is H, Li, Na, K, Rb, Cs, Mg, Ca, Sr or Ba and x is 1 or 2.

4. Process according to any one of the preceding claims, **characterized in that** the fluorination stage b) is carried out, in the liquid phase, in the presence of a polar aprotic solvent and of a fluorinating agent of formula A^{x+}Fₓ in which A is Li, Na, K, Mg or Ca and x is 1 or 2; advantageously, the polar aprotic solvent is chosen from the group consisting of an ether, an amide, an amine, a sulfoxide, a ketone, a nitrile or an ester.

5. Process according to the preceding claim, **characterized in that** the polar aprotic organic solvent has a boiling point of greater than 100°C at atmospheric pressure.

6. Process according to any one of Claims 1 to 3, **characterized in that** the fluorination stage b) is carried out in the gas phase in the presence of a catalyst and of HF.

7. Process according to any one of Claims 1 to 3, **characterized in that** the fluorination stage b) is carried out in the liquid phase in the presence of HF, advantageously in the presence of a catalyst based on metal halides or on an ionic liquid.

8. Process according to any one of the preceding claims, **characterized in that** hydrochloric acid is also produced during the fluorination stage, said second stream produced in stage b) comprising 1,1,2,3,4,4-hexafluorobutane, hydrochloric acid and optionally the unreacted fluorinating agent; advantageously, the second stream obtained in stage b) is separated into a first flow comprising hydrochloric acid and optionally the unreacted fluorinating agent and into a second flow comprising 1,1,2,3,4,4-hexafluorobutane.

9. Process according to any one of the preceding claims, **characterized in that** the hexafluorobutadiene formed in stage c) is recovered and subjected to a distillation stage.

10. Process according to any one of the preceding claims, **characterized in that** stage a) is carried out in the presence of hydrogen.

11. Process according to any one of the preceding claims, **characterized in that** stage a) is carried out in the gas phase at a temperature greater than the boiling point of hexachlorobutadiene.

12. Process according to any one of the preceding claims, **characterized in that** stage c) is carried out by i) chlorination of said second stream comprising 1,1,2,3,4,4-hexafluorobutane and then ii) dechlorination of the product obtained in i) in the presence of a zinc-comprising metal reactant.

13. Process according to the preceding claim, **characterized in that** the product obtained in i) is 1,2,3,4-tetrachloroperfluorobutane.
